# EUROPÄISCHE PATENTSCHRIFT

(11) **EP 3 357 524 B1**
(45) Veröffentlichungstag und Bekanntmachung des Hinweises auf die Patenterteilung: **02.10.2019**
(21) Anmeldenummer: 18151123.9
(22) Anmeldetag: 11.01.2018
(51) Int. Cl.: A61M 1/36

(54) **LUFTABSCHEIDER MIT ZWANGSZIRKULATION**
AIR SEPARATOR WITH FORCED CIRCULATION
SÉPARATEUR D'AIR À CIRCULATION FORCÉE

(30) Priorität: 03.02.2017 DE 102017102175
(43) Veröffentlichungstag der Anmeldung: 08.08.2018
(73) Patentinhaber: B. Braun Avitum AG, 34212 Melsungen (DE)
(72) Erfinder: Ritter, Kai-Uwe, 34212 Melsungen (DE)
(74) Vertreter: Winter, Brandl, Fürniss, Hübner, Röss, Kaiser, Polte - Partnerschaft mbB

(56) Entgegenhaltungen:
- EP-A1- 0 803 273
- EP-A1- 2 468 321
- DE-A1-102009 024 465
- US-A- 5 632 894

## Beschreibung

Die vorliegende Erfindung betrifft einen Luftabscheider einer Flüssigkeit handhabenden Maschine vorzugsweise einer extrakorporalen Blutbehandlungsmaschine wie Dialysemaschine mit einem, einem Flüssigkeitseinlass einer Luftabscheidekammer unmittelbar nachgeordneten Strömungsleitelement zur Erzeugung/Unterstützung einer Zirkulationsbewegung (Wirbel) der in die Luftabscheidekammer einströmenden Flüssigkeit.

### Hintergrund der Erfindung

Flüssigkeit handhabende Maschinen machen häufig die Anordnung von Luftabscheidern erforderlich, um die Funktionsfähigkeit der Maschine selbst und/oder eines nachgeordneten Verbrauchers/Empfängers der Flüssigkeit zu gewährleisten. Insbesondere bei medizinischen Maschinen wie extrakorporalen Blutbehandlungsmaschinen oder Herz-Lunge-Maschinen, die dafür vorgesehen sind, einem (menschlichen) Körper Blut als die zu handhabende Flüssigkeit zu entnehmen und wieder zuzuführen, ist es wichtig, sogenannte Mikroblasen im Blut als ein Begleiteffekt der extrakorporalen Blutbehandlung zu reduzieren, bevor das Blut in den Körper zurückgeführt wird.

So ist es derzeit in der Diskussion, ob diese Mikroblasen überhaupt als gesundheitsschädlich einzustufen sind. In der aktuellen Ausgabe der Norm 60601-2-16 wird deshalb aus Sicherheitsgründen auch erstmals ein oberer Grenzwert für Mikroblasen im Blut angegeben.

Die Entwicklung medizinischer, Flüssigkeit (Blut) handhabender Maschinen hat demzufolge darauf reagiert und u.a. Verfahren zur Erfassung von Mikroblasen in einem Bereich von 10-500µm Blasengröße beispielsweise auf der Basis eines gepulsten Ultraschall-Doppler-Systems entwickelt. Im Ergebnis wurde festgestellt, dass aus einem Körper entnommene sowie wieder zurückgeführte Flüssigkeit (Blut) infolge der extrakorporalen Behandlung mit Luftblasen angereichert ist, die demnach ebenfalls in den Patientenkörper geleitet werden, ohne dass hierbei ein Alarm ausgelöst wird.

Derartige Mikroblasen könnten vermutlich die Ursache von Kleinstembolien sein, welche innere Organe nachhaltig schädigen können.

Die US 5,632,894 offenbart einen Blutfilter mit einem Flüssigkeitseinlass der Blut aufwärts in die Kammer liefert. Dabei fließt das Blut auf der kreisrunden Passage bis zum Ende der Passage, die über dem Flüssigkeitseinlass liegt.

Die EP 2 468 321 A1 offenbart eine Kammer für ein Blutbehandlungssystem in dem an den Enden der Rohrstücke für den Bluteinlass, nicht nach unten weisende Endbereiche ausgeformt sind.

Die DE 10 2009 024 456 A1 offenbart einen Luftabscheider mit einer Luftabscheidekammer, mit wenigstens einem Einströmkanal, durch den das Fluid in die Luftabscheidekammer einströmen kann und mit einem Abströmkanal durch welchen das Fluid ausströmen kann.

Die EP 0 803 273 A1 offenbart eine Vorrichtung zum Abscheiden von Gasblasen aus Flüssigkeit, insbesondere Blut mit einem Strömungsleitbauteil, das einen rotationssymmetrischen Grundkörper aufweist, mit Leitschaufeln die sich im Wesentlichen tangential zur Wandung der Kammer erstrecken.

Es ist daher ein grundsätzliches Bestreben bei der Entwicklung von Flüssigkeit handhabenden, medizinischen Maschinen, wie extrakorporale Blutbehandlungsmaschinen, die Zahl an in einen Patientenkörper rückgeführten Mikroblasen zu reduzieren, um das Patientenrisiko zu verringern.

### Stand der Technik

Aus dem Stand der Technik sind Luftabscheider bekannt, die (Mikro-)Luftblasen aus einer Flüssigkeit abscheiden können und die auch für den Einsatz in der Medizin im Fall von Flüssigkeit handhabenden Maschinen wie extrakorporale Blutbehandlungsmaschinen (Dialysemaschinen) geeignet sind.

Ein solcher Luftabscheider hat in der Regel eine zumindest abschnittsweise trichter- oder kelchförmige sowie aufrecht stehende Luftabscheidekammer mit einem oberen Flüssigkeitseinlass (Bluteinlass) und einem unteren Flüssigkeitsauslass (Blutauslass), wobei ferner ein oberer Luftauslass vorgesehen ist. Insbesondere der Flüssigkeitseinlass ist so ausgebildet und ausgerichtet, dass daraus ausströmende Flüssigkeit in eine, eine allgemein axiale Flussrichtung überlagernde Zirkulationsbewegung längs des Gehäuseumfangs versetzt wird, wodurch auf die Flüssigkeit eine Zentrifugalkraft ausgeübt wird. Diese bewirkt, dass die Flüssigkeit innerhalb der Luftabscheidekammer radial nach Außen gedrückt wird, wohingegen darin enthaltene Luftblasen vornehmlich in der Mitte (nahe zur Gehäuse-Längsachse) verbleiben und in dem Gehäuse in Richtung hin zum Luftauslass aufsteigen können.

Um diese Zirkulation der einströmenden Flüssigkeit zu erhalten, wird der Flüssigkeitseinlass längs/tangential des Gehäuseumfangs bis zu 90° zur Gehäuselängsachse ausgerichtet, wodurch die Einströmung der Flüssigkeit maximal 90° zur Gehäuselängsachse umgelenkt werden und damit bestenfalls horizontal aus dem Flüssigkeitseinlass austreten kann.

Diese Technik scheidet Luftblasen vor allem bei niedrigen Flüssen gut ab. Bei höheren Flüssen reicht die Verweilzeit der Flüssigkeit in der Luftabscheidekammer aber häufig nicht mehr aus, um den Luftblasen genug Zeit einzuräumen, in Richtung hin zum Luftauslass aufzusteigen. Die Wirkung der Flüssigkeitszirkulation (Strudel) ist zwar immer noch vorhanden und unterstützt die Abscheidung der Mikroluftblasen, sie ist aber insbesondere bei höheren Flüssen deutlich begrenzt.

### Kurzbeschreibung der Erfindung

In Anbetracht dieser Problematik ist es die Aufgabe der vorliegenden Erfindung, einen Luftabscheider der vorstehend beschriebenen Gattung bereit zu stellen, dessen Luftabscheidewirkung insbesondere bei höheren Flüssen gegenüber dem bekannten Stand der Technik verbessert ist.

Diese Aufgabe wird durch einen Luftabscheider mit den Merkmalen des Patentanspruchs 1 gelöst. Vorteilhafte Ausgestaltungen der Erfindung sind Gegenstand der Unteransprüche.

Der Kern der vorliegenden Erfindung besteht demzufolge darin, dem zu einer Luftabscheidekammer hinführenden Flüssigkeitseinlass eines Luftabscheiders ein Strömungsleitelement (unmittelbar) nachzuordnen, mittels dem die einströmende Flüssigkeit in eine Strömungsrichtung zumindest längs/tangential des Kammerumfangs gezwungen wird. Durch das Strömungsleitelement kann die Flüssigkeit über einen gegenüber dem bekannten Stand der Technik längeren Strömungsweg auf einer vordefinierten Spiralbahn geführt werden, um so höhere Zentrifugalkräfte und auch längere Verweilzeiten der Flüssigkeit in der Luftabscheidekammer zu erreichen. Daraus resultiert eine gegenüber dem bekannten Stand der Technik verbesserte Abscheidung von (Mikro-)Luftblasen beispielsweise im Blut - auch bei höheren Flüssen. Darüber hinaus hat es sich gezeigt, dass ein solches Strömungsleitelement im Fall einer nicht ordnungsgemäßen Betriebsweise vorteilhaften Einfluss auf die Funktion des Luftabscheiders hat. Sollte nämlich der Füllstand der Luftabscheidekammer auf einen (unerwünscht) niedrigen Wert absinken, tropft die aus dem Flüssigkeitsauslass ausströmende Flüssigkeit (Blut) nicht mehr einfach auf die Oberfläche der in der Kammer noch befindlichen Flüssigkeit sondern wird noch ein Stück nach unten, sowie an der Kammerwand entlang geführt, um dann an der Kammerwand nach unten zu rinnen. Dadurch wird die in diesem Fall unerwünschte Erzeugung von Luftblasen und Schaumbildung infolge unkontrolliertem Auftreffen von Flüssigkeitstropfen auf die Flüssigkeitsoberfläche vermieden bzw. verringert.

Gemäß einem bevorzugten, ggf. unabhängig zu beanspruchenden Aspekt der vorliegenden Erfindung ist es vorgesehen, dass das Strömungsleitelement zumindest abschnittsweise die Querschnittsform einer Rinne hat. Dadurch wird eine bessere Zwangsführung der einströmenden Flüssigkeit erreicht.

Gemäß einem weiter bevorzugten, ggf. unabhängig zu beanspruchenden Aspekt der vorliegenden Erfindung ist es ferner vorgesehen, dass die Rinne in ihrem oberen Segment im Wesentlichen achsparallel zum Flüssigkeitseinlass vorzugsweise achsparallel zur Kammerachse verläuft sowie weiter vorzugsweise im Wesentlichen formschlüssig am Flüssigkeitseinlass anliegt. Auf diese Weise lassen sich Verwirbelungen im Bereich zwischen Flüssigkeitseinlass und Strömungsleitelement vermeiden/verringern, um so eine Schaumbildung der einströmenden Flüssigkeit zu verhindern.

Es ist vorgesehen, dass die Rinne sich spiralförmig längs der Umfangswand der Luftabscheidekammer in Richtung hin zum unteren Flüssigkeitsauslass erstreckt und an ihrem Auslauf in einem Winkel nach oben ausgerichtet ist. Dadurch kann die vom Strömungsleitelement zwangsgeführte Flüssigkeit innerhalb der Luftabscheidekammer sogar wieder nach oben beschleunigt und damit deren Verweilzeit in der Kammer verlängert werden.

Gemäß einem weiter bevorzugten, ggf. unabhängig zu beanspruchenden Aspekt der vorliegenden Erfindung ist es ferner vorgesehen, dass die Rinne an ihrem Auslauf unter einem Winkel von ca. 30° zur Kammerlängsachse nach oben ausgerichtet ist. Dieser Winkel hat sich insofern bewährt als dass hierdurch eine ausreichende Beschleunigung der Flüssigkeit nach oben erreichbar ist und gleichzeitig unerwünschte Turbulenzen insbesondere im Fall von Blut als die einströmende Flüssigkeit vermieden werden. Gemäß einem weiter bevorzugten, ggf. unabhängig zu beanspruchenden Aspekt der vorliegenden Erfindung ist es ferner vorgesehen, dass das Strömungsleitelement als separates Bauteil in die Luftabscheidekammer eingesetzt oder an die Luftabscheidekammer angefügt ist oder in die Wand der Luftabscheidekammer (innen- oder außenseitig) integriert ist.

Gemäß einem weiter bevorzugten, ggf. unabhängig zu beanspruchenden Aspekt der vorliegenden Erfindung ist es ferner vorgesehen, dass das Strömungsleitelement mindestens um ca. 30°, vorzugsweise um 90°-180° und maximal um 360° dem Kammerumfang folgt.

Gemäß einem weiter bevorzugten, ggf. unabhängig zu beanspruchenden Aspekt der vorliegenden Erfindung hat der Luftabscheider eine in Kammerumfangsrichtung verlaufende Abweiserwand, welche auf der dem Strömungsleitelement abgewandten Seite des Flüssigkeitseinlasses angeordnet ist und einen in Umfangsrichtung gesehen sanften Übergang zwischen der Kammerwand und dem als in Kammerachsrichtung vorragenden Stutzen ausgebildeten Flüssigkeitseinlass schafft. In anderen Worten ausgedrückt ragt der Flüssigkeitseinlass bevorzugt stutzen-/rohrförmig sowie achsparallel in die Luftabscheidekammer hinein, wobei sich ein oberes Segment der Rinne vorzugsweise zunächst ebenfalls achsparallel an den Flüssigkeitseinlass unmittelbar anschließt um dann nach Art einer Wasserrutsche fließend in eine Spiralform überzugehen. Somit kann die ausströmende sowie zwangsgeführte Flüssigkeit in Umfangsrichtung gesehen quasi hinter den Einlassstutzen gelangen, der dann als Strömungshindernis wirken würde. Um dies zu vermeiden ist die Abweiserwand vorgesehen, welche ein sanftes Übergehen der Kammerinnenwand zum Einlassstutzen bewirkt und damit die nach oben beschleunigten und dann zirkulierende Flüssigkeit am Einlassstutzen vorbei leitet.

Gemäß einem weiter bevorzugten, ggf. unabhängig zu beanspruchenden Aspekt der vorliegenden Erfindung ist es ferner vorgesehen, dass die Rinnenbreite zumindest in ihrem oberen Segment im Wesentlichen dem Durchmesser des Flüssigkeitseinlasses entspricht und vorzugsweise in Richtung hin zu ihrem unteren Segment breiter oder schmäler wird. Dadurch können unterschiedliche Strömungsgeschwindigkeiten mit unterschiedlichen Zentrifugalkräften realisiert und in Abhängigkeit der Kammergröße und Kammerform eingestellt werden.

### Kurzbeschreibung der Erfindung

Die Erfindung wird nachstehend anhand eines bevorzugten Ausführungsbeispiels unter Bezugnahme auf die begleitenden Figuren näher erläutert.

Es zeigen:
Fig. 1 die teiltransparente Seitenansicht eines oberen Abschnitts einer Luftabscheidekammer eines Luftabscheiders gemäß einem bevorzugten Ausführungsbeispiel der vorliegenden Erfindung,
Fig. 2 die teiltransparente Draufsicht der Luftabscheidekammer des Luftabscheiders gemäß der Fig. 1,
Fig. 3 die teiltransparente Seitenansicht des oberen Abschnitts der Luftabscheidekammer gemäß der Fig. 1 jedoch in einer um ca. 90° gedrehten Blickrichtung,
Fig. 4 die teiltransparente Seitenansicht des gesamten Luftabscheiders gemäß der vorliegenden Erfindung und
Fig. 5a bis 5c die prinzipielle Draufsichten von drei weiteren Abwandlungen eines Luftabscheiders gemäß der vorliegenden Erfindung.

### Figurenbeschreibung

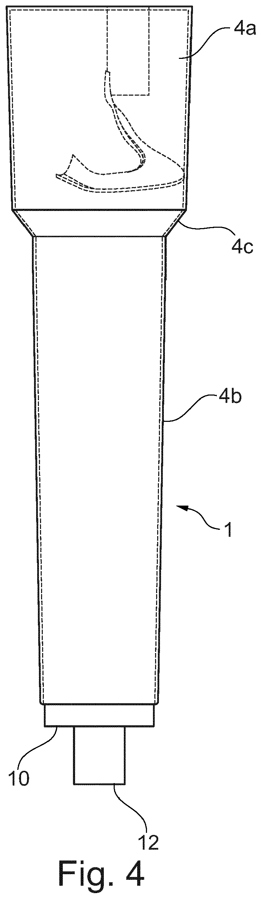

Der in der Fig. 4 in Seitenansicht dargestellte Luftabscheider 1 gemäß einem bevorzugten Ausführungsbeispiel der vorliegenden Erfindung ist dafür ausgebildet, um bevorzugt in den extrakorporalen Blutkreislauf einer extrakorporalen Blutbehandlungsmaschine wie Dialysemaschine allgemein bekannten Aufbaus eingesetzt zu werden. Bei dem erfindungsgemäßen Luftabscheider ist ein Strömungsleitelement 2 einem in eine (zylindrische/kelchförmige) Luftabscheidekammer 4 einmündenden Flüssigkeitseinlass 6 des Luftabscheiders 1 unmittelbar nachgeordnet, mittels dem eine einströmende Flüssigkeit, vorzugsweise Blut, in eine Strömungsrichtung mindestens längs/tangential des Kammerumfangs gezwungen wird.

Die in der Regel vertikal ausgerichtete Luftabscheidekammer 4 besteht vorliegend aus zwei im Wesentlichen zylinder- oder kelchförmigen Axialabschnitten 4a, 4b unterschiedlichen Durchmessers, wobei der obere Axialabschnitt mit großem Durchmesser 4a über eine trichterförmige Einschnürung 4c mit dem unteren Axialabschnitt mit kleinem Durchmesser 4b zusammengefügt ist.

Der obere Axialabschnitt 4a ist mittels eines Kammerdeckels 8 stirnseitig verschlossen, in dem der Flüssigkeitseinlass 6 vorzugsweise in Form eines in den oberen Axialabschnitt 4a der Luftabscheidekammer 4 vertikal/parallel zur Kammerachse ragenden (Einlass-)Stutzens sowie ein nicht näher dargestellter Luftauslass jeweils dezentral ausgebildet/angeordnet sind. Der untere Axialabschnitt 4b ist mittels eines Kammerbodens 10 stirnseitig verschlossen, in dem ein Flüssigkeitsauslass 12 zentral oder dezentral angeordnet ist.

Dieser Aufbau sowie die Dimensionierung der Luftabscheidekammer 4 einschließlich deren obere und untere Kammer-Axialabschnitte 4a, 4b insbesondere im Bereich der extrakorporalen Blutbehandlungsmaschine entsprechen dem aus dem Stand der Technik bestens bekannten Aufbau, sodass eine diesbezüglich nähere Angabe der einzelnen Bemaßungen an dieser Stelle überflüssig ist.

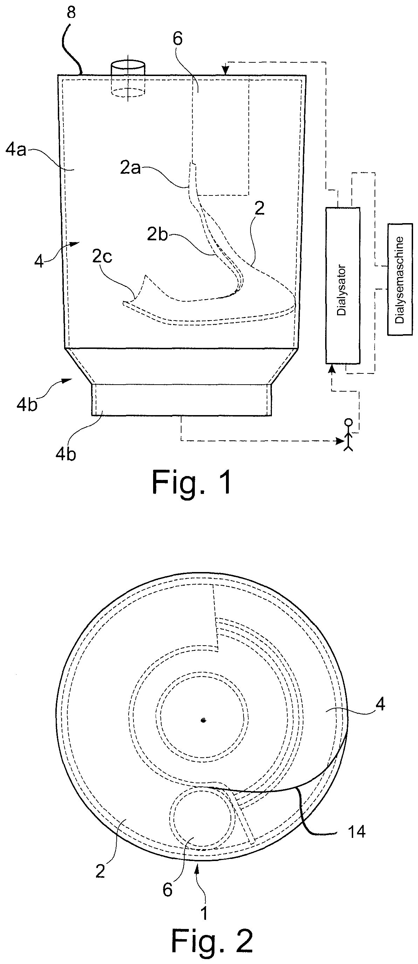

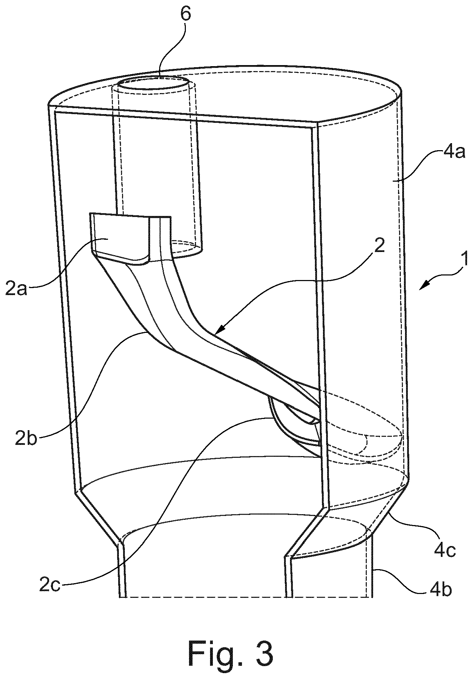

Das dem Flüssigkeitseinlass 6 nachgeordnete Strömungsleitelement 2 besteht in dem vorliegenden, bevorzugten Ausführungsbeispiel gemäß der Fig. 1 bis 3 aus einer spiralförmig längs der Kammerwand des oberen Axialabschnitts 4a verlaufenden Rutsche mit einer im Querschnitt U- bzw. rinnenartigen Form, die an Ihrem oberen/obersten Segment 2a (im Wesentlichen) vertikal/parallel zur Kammerachse verläuft, in einem Mittelsegment 2b fließend in einen (im Wesentlichen) horizontalen (senkrecht zur Kammerachse ausgerichteten) Verlauf übergeht und schließlich an ihrem unteren/untersten Segment 2c in einer Art Schanze endet, welche (im Wesentlichen) horizontal und bevorzugt geringfügig nach oben ausgerichtet ist.

Zur Vermeidung von Turbulenzen im Übergangsbereich zwischen dem Einlassstutzen 6 und der Rutsche 2 liegt deren oberes Segment 2a (möglichst) formschlüssig am Einlassstutzen 6 an, derart, dass Toträume vermieden werden.

Wie vorstehend bereits angedeutet wurde, kann die Rutsche 2 im Auslauf (Schanze) 2c bevorzugt nach oben gerichtet sein. Vorzugsweise ist die Schanze 2c ca. 30° bezüglich der Horizontalen nach oben gezogen.

Je nach Ausgestaltung kann die Rutsche 2 ein separates/zusätzliches in/an die Luftabscheidekammer 4 ein-/angefügtes Element sein, das ggf. bei Bedarf mit der Luftabscheidekammer 4 zusammengefügt oder demontiert werden kann. Alternativ hierzu ist es aber auch möglich, die Rutsche 2 in die Kammerwand zu integrieren, sodass die Luftabscheidekammer 4 und die Rutsche 2 aus einem einzigen Teil gefertigt werden kann.

Grundsätzlich hat die Rutsche 2 eine Spiralform mit ca. 30°, vorzugsweise 90-180° und maximal 360° Drehwinkel. Die axiale Erstreckung ist dabei der Axiallänge des oberen Kammer-Axialabschnitts 4a individuell angepasst und endet unmittelbar vor dem trichterförmigen Übergang 4c zum unteren Kammer-Axialabschnitt 4b.

Da die Schanze 2c der Rutsche 2 bevorzugt nach oben gerichtet ist, erfolgt eine Beschleunigung des einströmenden Bluts ebenfalls nach oben, derart, dass der in der Regel dezentral platzierte Einlassstutzen 6 vom nach oben beschleunigten Blut ggf. quer umströmt werden kann. In diesem Fall würde der Einlassstutzen 6 ein Strömungshindernis des nach oben beschleunigten, längs der Kammerwand zirkulierenden Bluts darstellen. Um dies zu vermeiden ist eine Abweiserwand 14 vorgesehen, welche einen sanften Übergang zwischen der Kammerwand und dem Einlassstutzen 6 auf der zur Rutsch 2 abgewandten Stutzenseite schafft und die das Blut gezielt am Einlassstutzen 6 vorbeiführt.

Der Querschnitt der Rutsche 2 ist bevorzugt dem Durchmesser des Einlassstutzens 6 angepasst und entspricht diesem (im Wesentlichen). Weiter bevorzugt bleibt der Rutschenquerschnitt über deren gesamte Länge konstant, kann sich aber in Richtung hin zur Schanze 2c aufweiten oder einschnüren.

In Funktion wird die Luftabscheidekammer 4 bevorzugt vollständig mit Blut gefüllt, d.h. bis nahezu zum oberen Kammerdeckel 8. Sollte die Kammer 4 in fehlerhafter Weise nur teilweise mit Blutgefüllt sein, ist darauf zu achten, dass der Blutpegel zumindest so hoch ist/bleibt, dass die Rutsche 2 und die innere Öffnung des Einlassstutzens 6 unterhalb der Blutoberfläche liegen. In diesem Zustand wird ein Großteil der einströmenden Flüssigkeit (Blut) zunächst einmal, wie gewünscht, nach oben beschleunigt und dabei in eine Zirkulationsbewegung versetzt, sodass sie zumindest ein bis mehrmals im Kreis wirbelt, wodurch die Abscheidung von Luftblasen ermöglicht wird.

Die Strömung im unteren Axialabschnitt der Luftabscheidekammer 4 ist vergleichsweise ungestört, ohne Turbulenzen und (im Wesentlichen) ohne Rotation. Dies bedeutet auch, dass der untere Axialabschnitt 4b des Luftabscheiders 1 gegenüber dem bekannten Stand der Technik verkürzt werden kann, wohingegen das Volumen des oberen Axialabschnitts 4a gegenüber dem bekannten Stand der Technik vergrößert sein kann.

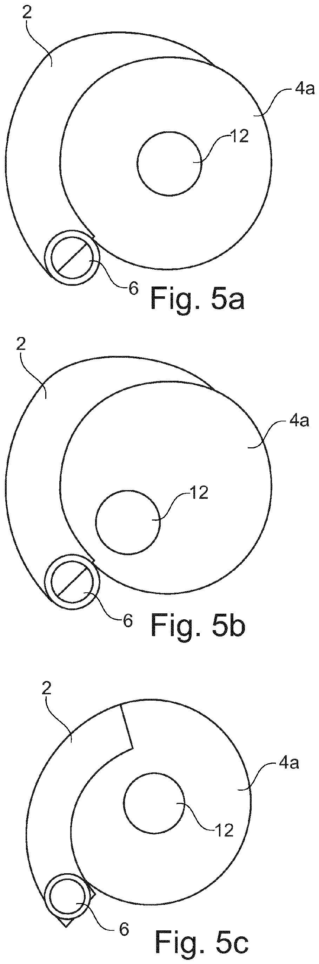

In den Fig. 5a-5c sind alternative Ausgestaltungen zum vorstehend beschriebenen bevorzugten Ausführungsbeispiel gezeigt, die jedoch dem gleichen Funktionsprinzip folgen, wie das bereits beschriebene Ausführungsbeispiel.

Demzufolge ist bei der Luftabscheidekammer 4 gemäß der Fig. 5a der Flüssigkeitseinlassstutzen 6 außerhalb der kreis-/zylinderförmigen Kontur des oberen Axialabschnitts 4a der Luftabscheidekammer 4 angebracht. Die dem Stutzen 6 unmittelbar nachgeordnete Rutsche 2 folgt dabei dem oberen Axialabschnitt 4a außenseitig um ca. 30°, bevorzugt 90-180°, maximal 360° vergleichbar zu dem vorstehend beschriebenen Ausführungsbeispiel. Der Rutschenquerschnitt kann über den Rutschverlauf konstant bleiben, sich aufweiten oder einschnüren. Im unteren Rutschensegment (Bereich der Schanze) 2c geht die Rutsche 2 in die kreisförmige Luftabscheidekammer 4 in deren oberen Axialabschnitt 4a über. Durch diese Ausgestaltung wird die Zirkulation innerhalb des in der Fig. 5a gezeigten Kreisbereichs der Luftabscheidekammer 2 gegenüber dem zuvor beschriebenen Ausführungsbeispiel ggf. verbessert, da der Einlassstutzen 6 kein Strömungshindernis mehr bildet.

Die Variante gemäß der Fig. 5b unterscheidet sich von der Variante gemäß der Fig. 5a in der Positionierung des Flüssigkeitsauslasses 12 im unteren Axialabschnitt 4b der Luftabscheidekammer 4. Demzufolge ist der Flüssigkeitsauslass 12 bei der Variante gemäß der Fig. 5a zentral (auf der Kammerachse) platziert, wohingegen der Flüssigkeitsauslass bei der Variante gemäß der Fig. 5b dezentral (in Richtung hin zur Kammerwand) versetzt ist, vorzugsweise in Richtung hin zur Umfangs-Positionierung des Flüssigkeitseinlasses 6.

Schließlich zeigt die Fig. 5c eine dritte Variante des erfindungsgemäßen Luftabscheiders 1, wonach der Flüssigkeitseinlassstutzen 6 ebenfalls außerhalb der im Querschnitt kreisförmigen Luftabscheidekammer 4 angebracht ist und die Rutsche 2 der Kontur des oberen Axialabschnitts 4a der Kammer 4 zunächst außen folgt, sich dann aber in ganzer Breite in den oberen Axialabschnitt 4a hineindreht.

Wie in der Fig. 5c dargestellt ist, folgt die Rutsche 2 danach über eine bestimmte Winkelstrecke der Kammerkontur an deren Kammerwand-Innenseite, um schließlich in der eingangs beschriebenen Schanze 2c zu enden. Auch bei dieser Variante kann die Rutsche 2 in Ihrem Querschnitt konstant bleiben, sich erweitern oder einschnüren.

## Patentansprüche

1. Luftabscheider einer extrakorporalen Blutbehandlungsmaschine wie Dialysemaschine mit einem, einem oberen Flüssigkeitseinlass (6) einer Luftabscheidekammer (4) unmittelbar nachgeordneten Strömungsleitelement (2) zur Erzeugung oder Unterstützung einer Zirkulationsbewegung der in die Luftabscheidekammer (4) einströmenden sowie in Richtung zu einem unteren Flüssigkeitsauslass (12) der Luftabscheidekammer (4) hin strömenden Flüssigkeit, wobei das Strömungsleitelement (2) zumindest abschnittsweise die Querschnittsform einer Rinne hat, und **gekennzeichnet dadurch, dass** die Rinne (2) sich spiralförmig längs der Umfangswand der Luftabscheidekammer (4) in Richtung hin zum unteren Flüssigkeitsauslass (12) erstreckt und an ihrem Auslauf (2c) in einem Winkel nach oben ausgerichtet ist.

2. Luftabscheider nach Anspruch 1, **dadurch gekennzeichnet, dass** die Rinne (2) in ihrem oberen Segment (2a) im Wesentlichen achsparallel zum Flüssigkeitseinlass (6) anliegt

3. Luftabscheider nach Anspruch 1, **dadurch gekennzeichnet, dass** die Rinne (2) an ihrem Auslauf (2c) unter einem Winkel von ca. 30° zur Kammerlängsachse nach oben ausgerichtet ist.

4. Luftabscheider nach einem der vorstehenden Ansprüche, **dadurch gekennzeichnet, dass** das Strömungsleitelement (2) als separates Bauteil in die Luftabscheidekammer (4) eingesetzt ist oder in die Wand der Luftabscheidekammer (4) integriert ist.

5. Luftabscheider nach einem der vorstehenden Ansprüche, **dadurch gekennzeichnet, dass** das Strömungsleitelement (2) mindestens um ca. 30 und maximal um 360° dem Kammerumfang spiralförmig folgt.

6. Luftabscheider nach einem der vorstehenden Ansprüche, **gekennzeichnet durch** eine in Kammerumfangsrichtung verlaufende Abweiserwand (14), welche auf der dem Strömungsleitelement (2) abgewandten Seite des Flüssigkeitseinlasses (6) angeordnet ist und einen, in Umfangsrichtung gesehen, sanften Übergang zwischen der Kammerwand und dem als in Kammerachsrichtung vorragenden Stutzen ausgebildeten Flüssigkeitseinlass (6) schafft.

7. Luftabscheider nach einem der vorstehenden Ansprüche, **dadurch gekennzeichnet, dass** die Rinnenbreite zumindest in ihrem oberen Segment (2a) im Wesentlichen dem Durchmesser des Flüssigkeitseinlasses (6) entspricht.

## Claims

1. An air separator of an extracorporeal blood treatment machine such as a dialysis machine, comprising a flow conducting element (2) arranged directly downstream of an upper fluid inlet (6) of an air separating chamber (4) for generating or backing a circulating movement of the fluid flowing into the air separating chamber (4) and flowing toward a lower fluid outlet (12) of the air separating chamber (4), wherein the flow conducting element (2) at least in portions takes the cross-sectional shape of a groove, and
**characterized in that**
the groove (2) extends in spiral shape along the peripheral wall of the air separating chamber (4) toward the lower fluid outlet (12) is orientated upwards at an angle at its runout (2c).

2. The air separator according to claim 1, **characterized in that** in its upper segment (2a) the groove (2) extends substantially axially in parallel to the fluid inlet (6).

3. The air separator according to claim 1, **characterized in that** at its runout (2c) the groove (2) is orientated upwards at an angle of about 30° relative to the longitudinal chamber axis.

4. The air separator according to one of the preceding claims, **characterized in that** the flow conducting element (2) is inserted in the air separating chamber (4) as a separate component or is integrated in the wall of the air separating chamber (4).

5. The air separator according to one of the preceding claims, **characterized in that** the flow conducting element (2) follows the chamber periphery in spiral shape at least by about 30° and maximally by 360°.

6. The air separator according to one of the preceding claims, **characterized by** a deflector wall (14) extending in the peripheral chamber direction which is arranged on the side of the fluid inlet (6) facing away from the flow conducting element (2) and, when viewed in the peripheral direction, creates a smooth transition between the chamber wall and the fluid inlet (6) in the form of a nozzle protruding in the axial chamber direction.

7. The air separator according to one of the preceding claims, **characterized in that** the groove width substantially corresponds, at least in its upper segment (2a), to the diameter of the fluid inlet (6).

## Revendications

1. Séparateur d'air d'une machine de traitement extracorporel du sang telle qu'une machine de dialyse, avec un élément de guidage d'écoulement (2) disposé directement en aval d'une entrée de liquide (6) supérieure d'une chambre de séparation d'air (4), servant à générer ou à assister un mouvement de circulation du liquide affluant dans la chambre de séparation d'air (4) et s'écoulant également en direction d'une sortie de liquide (12) inférieure de la chambre de séparation d'air (4), dans lequel l'élément de guidage d'écoulement (2) a au moins par endroits la forme en section transversale d'une goulotte, et **caractérisé en ce que** la goulotte (2) s'étend en forme de spirale le long de la paroi périphérique de la chambre de séparation d'air (4) en direction de la sortie de liquide (12) inférieure et est orientée au niveau de son évacuation (2c) vers le haut selon un angle.

2. Séparateur d'air selon la revendication 1, **caractérisé en ce que** la goulotte (2) repose dans son segment supérieur (2a) sensiblement de manière axialement parallèle par rapport à l'entrée de liquide (6).

3. Séparateur d'air selon la revendication 1, **caractérisé en ce que** la goulotte (2) est orientée au niveau de son évacuation (2c) vers le haut selon un angle d'environ 30° par rapport à l'axe longitudinal de chambre.

4. Séparateur d'air selon l'une quelconque des revendications précédentes, **caractérisé en ce que** l'élément de guidage d'écoulement (2) est inséré en tant que composant séparé dans la chambre de séparation d'air (4) ou est intégré dans la paroi de la chambre de séparation d'air (4).

5. Séparateur d'air selon l'une quelconque des revendications précédentes, **caractérisé en ce que** l'élément de guidage d'écoulement (2) suit en forme de spirale au moins sur environ 30 et au maximum sur 360° la périphérie de chambre.

6. Séparateur d'air selon l'une quelconque des revendications précédentes, **caractérisé par** une paroi déflectrice (14) s'étendant dans la direction périphérique de chambre, laquelle est disposée sur le côté, opposé à l'élément de guidage d'écoulement (2), de l'entrée de liquide (6) et crée, dans une vue dans la direction périphérique, une transition douce entre la paroi de chambre et l'entrée de liquide (6) réalisée en tant que tubulure dépassant dans la direction axiale de chambre.

7. Séparateur d'air selon l'une quelconque des revendications précédentes, **caractérisé en ce que** la largeur de goulotte correspond au moins dans son segment supérieur (2a) sensiblement au diamètre de l'entrée de liquide (6).
